# EUROPEAN PATENT SPECIFICATION

(11) **EP 1 309 705 B1**
(45) Date of publication and mention of the grant of the patent: **15.02.2012**
(21) Application number: 01962303.2
(22) Date of filing: 25.07.2001
(51) Int. Cl.: C12N 15/62

(54) **MULTIVALENT TARGET BINDING PROTEIN**
MULTIVALENTES ZIELBINDENDES PROTEIN
PROTEINE DE LIAISON CIBLE MULTIVALENTE

(30) Priority: 25.07.2000 US 220782 P
(43) Date of publication of application: 14.05.2003
(73) Proprietor: Immunomedics Inc., Morris Plains, NJ 07950 (US)
(72) Inventor: LEUNG, Shui-on, Hong Kong Inst. Biotechnology Ltd, Shatin, N.T. (HK); HANSEN, Hans, Morris Plains, NJ 07950 (US)
(74) Representative: Bohmann, Armin K.
(86) International application number: PCT/US2001/041386
(87) International publication number: WO 2002/008293

(56) References cited:
- WO-A-00/06605
- WO-A-02/02781
- WO-A-94/09131
- WO-A-99/37791
- SCHOONJANS R ET AL: "EFFICIENT HETERODIMERIZATION OF RECOMBINANT BI- AND TRISPECIFIC ANTIBODIES" BIOSEPARATION, KLUWER ACADEMIC PUBLISHERS, DORDRECHT, NL, vol. 9, no. 3, 2000, pages 179-183, XP001012944 ISSN: 0923-179X
- SCHOONJANS R ET AL: "Fab chains as an efficient heterodimerization scaffold for the production of recombinant bispecific and trispecific antibody derivatives." JOURNAL OF IMMUNOLOGY (BALTIMORE, MD.: 1950) UNITED STATES 15 DEC 2000, vol. 165, no. 12, 15 December 2000 (2000-12-15), pages 7050-7057, XP002207879 ISSN: 0022-1767
- SCHOONJANS R ET AL: "A new model for intermediate molecular weight recombinant bispecific and trispecific antibodies by efficient heterodimerization of single chain variable domains through fusion to a Fab-chain." BIOMOLECULAR ENGINEERING. NETHERLANDS JUN 2001, vol. 17, no. 6, June 2001 (2001-06), pages 193-202, XP002207880 ISSN: 1389-0344
- ZUO Z ET AL: "An efficient route to the production of an IgG-like bispecific antibody." PROTEIN ENGINEERING. ENGLAND MAY 2000, vol. 13, no. 5, May 2000 (2000-05), pages 361-367, XP002207881 ISSN: 0269-2139
- MÜLLER K ET AL: "The first constant domain (CH1 and CL) of an antibody used as heterodimerization domain for bispecific miniantibodies" FEBS LETTERS, ELSEVIER SCIENCE PUBLISHERS, AMSTERDAM, NL, vol. 422, no. 2, 30 January 1998 (1998-01-30), pages 259-264, XP002135067 ISSN: 0014-5793
- DATABASE BLOOD LNKD-PUBMED: 19710501 18, VOL. 114, 29 October 2009 ROSSI EDMUND A ET AL: 'CD20-targeted tetrameric interferon-alpha, a novel and potent immunocytokine for the tharapy of B-cell lymphomas.pages: 3864-3871'
- ROSSI EDMUND A ET AL: "CD20-targeted tetrameric interferon-alpha, a novel and potent immunocytokine for the therapy of B-cell lymphomas.", BLOOD 29 OCT 2009 LNKD- PUBMED:19710501, vol. 114, no. 18, 29 October 2009 (2009-10-29), pages 3864-3871, ISSN: 1528-0020
- WANG ZHONGMIN ET AL: "Blood pharmacokinetics of various monoclonal antibodies labeled with a new trifunctional chelating reagent for simultaneous conjugation with 1,4,7,10-tetraazacyclododecane-N,N',N'',N' ''-tetraacetic acid and biotin before radiolabeling.", CLINICAL CANCER RESEARCH : AN OFFICIAL JOURNAL OF THE AMERICAN ASSOCIATION FOR CANCER RESEARCH 1 OCT 2005 LNKD- PUBMED:16203818, vol. 11, no. 19 Pt 2, 1 October 2005 (2005-10-01), pages 7171s-7177s, ISSN: 1078-0432

## Description

### BACKGROUND OF THE INVENTION

### Field of the Invention

The present invention relates to a multivalent target binding protein, and methods of using the multivalent target binding protein for treatment and detection of tumors and infectious lesions.

### Related Art

Multivalent target binding proteins are useful for treating or detecting tumors and other diseases. For instance, a multivalent target binding protein may bind to both a tumor antigen and a cytotoxic agent, and can be used for delivery of radionuclides, drugs, toxins or other cytotoxic agents to tumor cells.

It is desirable to increase the valency of a target binding protein. The increased valency can improve the avidity of the target binding protein to its target, and therefore increase the specificity and safety of a treatment. A target binding protein with increased valency can also be useful for simultaneously delivering different cytotoxic agents to a single target, or for delivering a cytotoxic agent to different targets. A multivalent targeting binding protein can further be used to recruit different immune effector cells to a single target cell, and thus trigger an enhanced immune response against the target cell.

Efforts have been made to produce multivalent target binding proteins which have at least three different target binding sites. For example, multivalent target binding proteins have been made by cross-linking several Fab-like fragments via chemical linkers. *See* US Patent No. 5,262,524. *See also* US Patent No. 5,091,542 and Landsdorp, et al., "Cyclic Tetramolecular Complexes Of Monoclonal Antibodies: A New Type Of Cross-linking Agent," Euro. J. Immunol., 16: 679-83 (1986). Multivalent target binding proteins also have been made by covalently linking several single chain Fv molecules (scFv) to form a single polypeptide. *See* US Patent No. 5,892,020. A multivalent target binding protein which is basically an aggregate of scFv molecules has been disclosed in US Patent Nos. 6,025,165 and 5,837,242. A trivalent target binding protein comprising three scFv molecules has been described in Krott et al., "Single-chain Fv Fragment of Anti-Neuraminidase Antibody NC 10 Containing Five- and Ten-Residue Linkers Form Dimers and Zero-Residue Linker A Trimer" Protein Engineering, 10(4): 423-433 (1997). Other binding proteins and methods for making them are described in WO99/37791 and Schoonjans et al. (Bioseparation, 2000, 9(3):179-183; J. Immunol. 2000, 165(12):7050-7057 and Biomolecular Engineering, 2001, 17(6):193-202) disclose scFv molecules conjugated through a CL or CH1 region by a linker to a VH or VL region and a second polypeptide comprising a scFv and a CL or CH1 region and a VL or VH region, and that the molecules have a disulfide bond in the extra amino acid sequence, which is in the constant region. WO94/09131 discloses the fusion of an Fd fragment and a light chain to scFv. However, the above mentioned methods either lack reproducibility or lack the capability to produce a protein having different, pre-selected target-binding specificities.

The present invention discloses a novel form of multivalent target binding proteins having different, pre-selected binding specificities and the method for making which is reproducible. The multivalent binding protein of the present invention comprises two polypeptides which associate to form at least three target binding sites. The present invention also provides a new way for making multivalent target binding proteins.

### SUMMARY OF THE INVENTION

It is therefore an object of the present invention to provide a novel form of target binding protein that comprises at least three target binding sites as defined in the claims.

It is also an object of the present disclosure to provide methods of using the multivalent binding protein of the present invention for treating and detecting tumors or infectious lesions.

In achieving these objects, there has been provided, a target binding protein comprising three target binding sites, wherein said protein comprises a first polypeptide comprising a first single chain Fv molecule covalently linked to a first immunoglobulin-like domain, and a second polypeptide comprising a second single chain Fv molecule covalently linked to a second immunoglobulin-like domain, wherein said first single chain Fv molecule forms a first target binding site and said second single chain Fv molecule forms a second target binding site, and wherein said first immunoglobulin-like domain associates with said second immunoglobulin-like domain to form a third target binding site. Alternatively, the first and second single chain Fv molecules may associate together to form two binding sites, with the first and second immunoglobulin-like domains associating to form a third binding site.

In accordance with another aspect of the present disclosure, the first single chain Fv molecule and the first immunoglobulin-like domain are covalently linked via a first extra amino acid sequence, and the second single chain Fv molecule and the second immunoglobulin-like domain are covalently linked via a second extra amino acid sequence. The first extra amino acid sequence associates with said second extra amino acid sequence, preferably via covalent interactions, and more preferably via at least one disulfide bond.

In another aspect of the present disclosure, the first immunoglobulin-like domain comprises an immunoglobulin light chain variable region domain or a derivative thereof, which is covalently linked to the first scFv molecule via an immunoglobulin light chain constant region domain or a derivative thereof, and the second immunoglobulin-like domain comprises an immunoglobulin heavy chain variable region domain or a derivative thereof, which is covalently linked to the second scFv molecule via an immunoglobulin heavy chain constant region domain or a derivative thereof.

In yet another aspect of the present disclosure, the first single chain Fv molecule and the immunoglobulin light chain constant region domain are covalently linked via a first peptide linker which preferably comprises the amino acid sequence EPKSADKTHTCPPCPGGGS, and the second single chain Fv and the immunoglobulin heavy chain constant region domain are covalently linked via a second peptide linker which preferably comprises the amino acid sequence EPKSCDKTHTCPPCPGGGS.

In accordance with another aspect of the present invention, two of the three target binding sites have different target binding specificities.

In yet another aspect of the present invention, two of the three target binding sites have the same target binding specificity.

There has also been provided, in accordance with another aspect of the present disclosure, that either the first or second polypeptide is covalently linked to additional amino acid residues at its N- or C-terminus. The additional amino acid residues preferably comprise a peptide tag, a signal peptide, an enzyme, a cytokine, a toxin, a drug, a cytotoxic protein, or another functional protein.

In another aspect of the present disclosure, a carbohydrate chain is covalently linked to either the first or second polypeptide via a N-glycosylation recognition sequence engineered to the first or second polypeptide. The carbohydrate chain is preferably further linked to a drug, a radioactive compound, a chelate, an enzyme, a toxin, a cytokine, a cytotoxic protein, or another functional agent.

In accordance with yet another aspect of the present disclosure, a drug, a radioactive compound, a chelate, an enzyme, a toxin, a cytokine, a cytotoxic protein, or another functional agent is conjugated to the multivalent binding protein of the present via the side chain of the amino acid residues of the multivalent binding protein.

In accordance with another aspect of the present disclosure, one target binding site of the multivalent binding protein of the present invention binds to a toxin, a drug, a cytokine, a chelate, an enzyme, a radioactive compound, a cytotoxic protein or other functional agents, while the other two target binding sites bind to tumor antigens.

A multivalent target binding protein has also been provided, in accordance with one aspect of the present disclosure, wherein one target binding site of the multivalent protein binds to a tumor antigen and the other two target binding sites bind to the surface proteins of T cells or other effector cells.

There has been provided, in accordance with another aspect of the present disclosure a nucleic acid molecule which comprises a first polynucleotide encoding the first polypeptide of the multivalent binding protein and a second polynucleotide encoding the second polypeptide of the multivalent binding protein.

In yet another aspect of the present disclosure, there has been provided two nucleic acid molecules, one encoding the first polypeptide of the multivalent binding protein and the other encoding the second polypeptide of the multivalent binding protein. Additionally, the current disclosure provides vectors comprising the nucleic acids, and , in turn, host cells comprising these vectors.

There has also been provided, in accordance with another aspect of the present disclosure, a method of making the multivalent binding protein of the present invention.

In yet another aspect of the present disclosure, there has been provided a method of eliciting an enhanced immune response against a tumor comprising administering to a patient suffering from said tumor an effective amount of the multivalent target binding protein of the present invention, wherein one target binding site of the protein binds to the tumor, and the other two target binding sites bind to two different surface proteins on T cells or other effector cells.

In yet another aspect of the present disclosure, there has been provided a method of treating or detecting a tumor comprising administering to a patient suffering from said tumor an effective amount of the multivalent target binding protein of the present invention, wherein one target binding site of the protein binds to a toxin, a drug, a cytokine, a chelate, an enzyme, a radioactive compound, a cytotoxic protein or other functional agents, and the other two target binding sites bind to tumor antigens.

In yet another aspect of the present disclosure, there has been provided a method of treating or detecting a tumor comprising administering to a patient suffering from said tumor an effective amount of the multivalent target binding protein of the present invention, wherein at least one target binding site, and preferably three target binding sites, of the protein binds to tumor antigens, while a toxin, a drug, a cytokine, a chelate, an enzyme, a radioactive compound, a cytotoxic protein or another functional agent is conjugated to the protein.

In yet another aspect of the present disclosure, there has been provided a method of treating a tumor, using the target binding protein of the current invention.

### BRIEF DESCRIPTION OF THE DRAWINGS

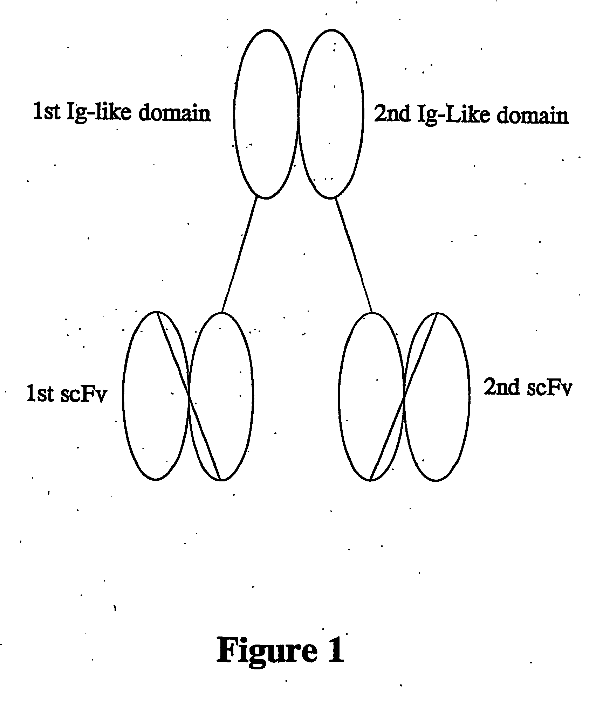

Figure 1 shows a schematic representation of a multivalent target binding protein which comprises two polypeptides. A first polypeptide comprises a first single chain Fv molecule (1st scFv) covalently linked to a first immunoglobulin-like domain (1st Ig-like domain). A second polypeptide comprises a second single chain Fv molecule (2nd scFv) covalently linked to a second immunoglobulin-like domain (2nd Ig-like domain).

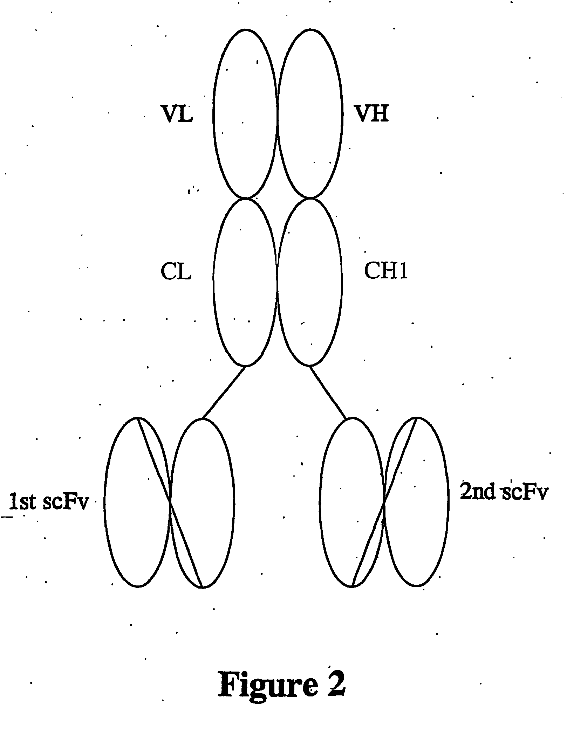

Figure 2 shows a schematic representation of a multivalent target binding protein which comprises two polypeptides. A first polypeptide comprises a first single chain Fv molecule (1st scFv) covalently linked to an immunoglobulin light chain fragment which comprises the constant region CL and the variable region VL. A second polypeptide comprises a second single chain Fv molecule (2nd scFv) covalently linked to an immunoglobulin heavy chain fragment which comprises the constant region CH1 and the variable region VH.

### DESCRIPTION OF SPECIFIC EMBODIMENTS

This invention provides a multivalent target binding protein comprising at least three target binding sites as defined by the claims. The three target binding sites can be directed to the same or different targets. The multivalent binding protein of the present disclosure comprises a first and a second polypeptide. The first polypeptide comprises a first single chain Fv molecule covalently linked to a first immunoglobulin-like domain which preferably is an immunoglobulin light chain variable region domain. The second polypeptide comprises a second single chain Fv molecule covalently linked to a second immunoglobulin-like domain which preferably is an immunoglobulin heavy chain variable region domain. The first and second single chain Fv molecules form two target binding sites, and the first and second immunoglobulin-like domains associate to form a third target binding site. Alternatively, the first and second single chain Fv molecules may associate together to form two binding sites, with the first and second immunoglobulin-like domains associating to form a third binding site. Preferably, the first single chain Fv molecule and the first immunoglobulin-like domain are covalently linked via a first extra amino acid sequence which preferably comprises an immunoglobulin light chain constant region domain, and the second single chain Fv molecule and the second immunoglobulin-like domain are covalently linked via a second extra amino acid sequence which preferably comprises an IgG1 immunoglobulin heavy chain constant region domain. More preferably, the first extra amino acid sequence and the second extra amino acid sequence associate with each other, preferably via covalent interactions such as disulfide bonds, so as to stabilize the association between the first and second polypeptides.

As used herein, the term "antibody" is used interchangeably with the term "immunoglobulin." The terms "domain" or "fragment" mean a portion of the amino acid sequence of a protein.

### Antibody Structure

There are at least five classes of human antibodies, each class having the same basic structure. The basic structure of an antibody is a tetramer, or a multimeric form thereof, composed of two identical heterodimers, each heterodimer consisting of a light chain with a molecular weight of about 25 kDa and a heavy chain with a molecular weight of about 50-77 kDa. For instance, Immunoglobulin G (IgG) consists of two identical heterodimers, while Immunoglobulin M (IgM) has five identical heterodimers. The two heterodimers of an IgG molecule are covalently linked via disulfide bonds. The light chain and the heavy chain of each heterodimer also are covalently linked via at least one disulfide bond.

Each light or heavy chain folds into several regions. Each region consists of approximately 110 amino acid residues, and has a conserved three-dimensional conformation. The light chain comprises one variable region (VL) and one constant region (CL). The heavy chain comprises one variable region (VH) and three constant regions (CH1, CH2 and CH3). The CH1 region and CH2 region of the heavy chain are linked by a hinge region. The VL and VH regions of an antibody are associated to form an antigen-binding site. This association primarily involves non covalent interactions. "Fv" denotes the structure formed by the association of VL and VH. The areas on an antigen that interact with the antigen-binding site are called epitopes. The epitopes fit into the conformational architecture of the antigen-binding site of an antibody, enabling the antibody to bind to the antigen. The interactions between the antigen and the antigen-binding site determines the specificity of an antibody.

The CL and CH1 regions of an antibody are associated via non covalent interactions. The CL region also is linked to the hinge region of the heavy chain via a disulfide bond. For example, Cys214 (Kabat's numbering) of the kappa type of light chain can form a disulfide bond with Cys233 (Kabat's numbering) of the hinge region of the heavy chain. For Kabat's numbering, see Kabat EA, Wu TT, Perry HM, Gottesman KS and Foeller C. (1991), Sequences of proteins of immunological interest (5th edition, US Dept. Health and Human Services, US Government Printing Office), which is hereby incorporated by reference. The association between the CL and CH1 regions, as well as the disulfide bond between the CL region and the hinge region, contribute to the stabilization of the three-dimensional structure of an antibody.

The variable regions (VL and VH) show considerable variability in structure and amino acid composition from one immunoglobulin molecule to another. The constant regions (CL, CH1, CH2 and CH3), however, show little variability. The term "variable" as used in this specification refers to the diverse nature of the amino acid sequences of the antibody heavy and light chain variable regions. Within the variable regions are found regions in which the amino acid sequence is extremely variable from one antibody to another. Three of these so-called "hypervariable" regions or "complementarity-determining regions" (CDR) are found in each variable region of the light or heavy chain. Each CDR is flanked by relatively conserved framework regions (FR). The FR are thought to maintain the structural integrity of the variable region. The CDRs of a light chain and the CDRs of a corresponding heavy chain form the antigen-binding site. The "hypervariability" of the CDRs accounts for the diversity of specificity of antibodies.

Cleavage of a naturally-occurring antibody molecule with the protease papain generates fragments which retain the antigen-binding site. These fragments, commonly known as Fab fragments, comprise the light chain (VL and CL) and a fragment of the heavy chain (VH, CH1 and part of the hinge region) of the antibody. The light chain and the fragment of the heavy chain are covalently linked via at least one disulfide bond.

Antibodies are members of the immunoglobulin superfamily of proteins. Members of this superfamily also include, but are not limited to, T cell receptors, CD2, CD4, CD8, and certain types of cell-cell adhesion molecules. See Molecular Biology of The Cell (2nd edition, Bruce Alberts et al., Garland Publishing, Inc., 1989), pp 1053-1054. The basic building block for members of the immunoglobulin superfamily proteins is termed an "immunoglobulin-like domain. An "immunoglobulin-like domain" consists of about 100 amino acids, folded into a characteristic sandwichlike structure made of two antiparallel beta sheets. *See id.* Each naturally occurring "immunoglobulin-like domain" is usually encoded by a separate *exon*. *See id.* A typical immunoglobulin-like domain includes the variable and constant region of an antibody.

### Single Chain Fv Molecule

A single chain Fv molecule (scFv) comprises a VL domain and a VH domain. The VL and VH domains associate to form a target binding site. These two domains are further covalently linked by a peptide linker (L). A scFv molecule is denoted as either VL-L-VH if the VL domain is the N-terminal part of the scFv molecule, or as VH-L-VL if the VH domain is the N-terminal part of the scFv molecule. Methods for making scFv molecules and designing suitable peptide linkers are described in US Patent No. 4,704,692, US Patent No. 4,946,778, R. Raag and M. Whitlow, "Single Chain Fvs." FASEB Vol 9:73-80 (1995) and R.E. Bird and B.W. Walker, "Single Chain Antibody Variable Regions," TIBTECH, Vol 9: 132-137 (1991). These references are incorporated herein by reference.

A single chain Fv molecule with the VL-L-VH configuration may associate with another single chain Fv molecule with the VH-L-VL configuration to form a bivalent dimer. In this case, the VL domain of the first scFv and the VH domain of the second scFv molecule associate to form one target binding site, while the VH domain of the first scFv and the VL domain of the second scFv associate to form the other target binding site.

### Multivalent Target Binding Protein

In one embodiment, a multivalent target binding protein is constructed by association of a first and a second polypeptide. *See* Figure 1. The first polypeptide comprises a first single chain Fv molecule covalently linked to a first immunoglobulin-like domain which preferably is an immunoglobulin light chain variable region domain. The second polypeptide comprises a second single chain Fv molecule covalently linked to a second immunoglobulin-like domain which preferably is an immunoglobulin heavy chain variable region domain. Each of the first and second single chain Fv molecules forms a target binding site, and the first and second immunoglobulin-like domains associate to form a third target binding site.

In a preferred embodiment, the first immunoglobulin-like domain comprises an antibody light chain variable region domain (VL domain) or a derivative thereof, and the second immunoglobulin-like domain comprises an antibody heavy chain variable region domain (VH domain) or a derivative thereof. The VL and VH domains may be synthetic domains constructed *in vitro* using techniques as described in WO 93/11236. The VL domain, or its derivative, associates with the VH domain or its derivative to form a functional target binding site. A domain is a derivative of another domain if the two domains have more than 50%, preferably more than 70%, most preferably more than 90%, amino acid sequence identity. "Amino acid sequence identify" has an art-recognized meaning and can be calculated using published techniques. *See* COMPUTATIONAL MOLECULAR BIOLOGY, Lesk, A. M., ed., Oxford University Press, New York, 1988; BIOCOMPUTING: INFORMATICS AND GENOME PROJECTS, Smith, D. W., ed., Academic Press, New York, 1993; COMPUTER ANALYSIS OF SEQUENCE DATA, PART I, Griffin, A. M., and Griffin, H. G., eds., Humana Press, New Jersey, 1994; SEQUENCE ANALYSIS IN MOLECULAR BIOLOGY, Von Heinje, G., Academic Press, 1987; and SEQUENCE ANALYSIS PRIMER, Gribskov, M. and Devereux, J., eds., M Stockton Press, New York, 1991. While there exist a number of methods to measure identity between two amino acid sequences, the term "identity" is well known to skilled artisans. *See* Carillo, H., and Lipton, D., SIAM J Applied Math (1988) 48:1073, which is hereby incorporated by reference. Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., SIAM J Applied Math (1988) 48:1073. Methods to determine identity and similarity are codified in computer programs. Preferred computer program methods to determine identity and similarity between two sequences include, but are not limited to, GCG program package (Devereux, J., et al., Nucleic Acids Research (1984) 12(1):387), BLASTP, BLASTN, FASTA (Atschul, S. F. et al., J. Mol. Biol. (1990) 215:403), and FASTDB (Brutlag et al., Comp. App. Biosci. (1990) 6:237-245).

A more preferred method for determining the best overall match, also referred to as a global sequence alignment, between a query sequence (for example, a sequence of the present invention) and a subject sequence can be determined using the FASTDB computer program based on the algorithm of Brutlag et al. (Comp. App. Biosci. (1990) 6:237-245). In a sequence alignment the query and subject sequences are either both nucleotide sequences or both amino acid sequences. The result of said global sequence alignment is in percent identity. Preferred parameters used in a FASTDB amino acid alignment are: Matrix=PAM 0, k-tuple=2, Mismatch Penalty=1, Joining Penalty=20, Randomization Group Length=zero, Cutoff Score= 1, Window Size=sequence length, Gap Penalty=5, Gap Size Penalty=0.05, Window Size=500 or the length of the subject amino acid sequence, whichever is shorter. If the subject sequence is shorter than the query sequence due to N- or C-terminal deletions, not because of internal deletions, a manual correction must be made to the results. This is because the FASTDB program does not account for N- and C-terminal truncations of the subject sequence when calculating global percent identity. For subject sequences truncated at the N-and C-termini, relative to the query sequence, the percent identity is corrected by calculating the number of residues of the query sequence that are N- and C-terminal of the subject sequence, which are not matched/aligned with a corresponding subject residue, as a percent of the total bases of the query sequence. Whether a residue is matched/aligned is determined by results of the FASTDB sequence alignment. This percentage is then subtracted from the percent identity, calculated by the above FASTDB program using the specified parameters, to arrive at a final percent identity score. This final percent identity score is what is used for the purposes of the present invention. Only residues N- and C-terminal to the subject sequence, which are not matched/aligned with the subject sequence, are considered for the purposes of manually adjusting the percent identity score. That is, only query residue positions outside the farthest N-and C-terminal residues of the subject sequence are considered. For example, a 90 amino acid residue subject sequence is aligned with a 100 residue query sequence to determine percent identity. The deletion occurs at the N-terminus of the subject sequence and, therefore, the FASTDB alignment does not show a matching/alignment of the first 10 residues at the N-terminus. The 10 unpaired residues represent 10% of the sequence (number of residues at the N- and C- termini not matched/total number of residues in the query sequence). Thus, 10% is subtracted from the percent identity score calculated by the FASTDB program. If the remaining 90 residues were perfectly matched, the final percent identity would be 90%. In another example, a 90 residue subject sequence is compared with a 100 residue query sequence. This time the deletions are internal deletions so there are no residues at the N- or C-termini of the subject sequence which are not matched/aligned with the query sequence. In this case the percent identity calculated by FASTDB is not manually corrected. Once again, only residue positions outside the N- and C-terminal ends of the subject sequence, as displayed in the FASTDB alignment, which are not matched/aligned with the query sequence are manually corrected for.

Whether a VL domain or its derivative is able to associate with a VH domain or its derivative to form a functional target binding site may be tested using M13 bacteriophage display. For example, the cDNA encoding the VL domain or its derivative and the DNA encoding the VH domain or its derivative may be ligated to form a scFv sequence. The scFv sequence thus formed may be subcloned into a M13 phage display vector. The affinity of the expressed scFv molecule to the desired target may then be determined using routine phage display techniques. For phage display techniques, see Phage display of peptides and proteins: A laboratory Manual (1996) (Eds. Kay, B., et al., Academic Press, San Diego); Dunn IS, Curr. Opin. Biotechnol., 7:547-553 (1996); Smith GD and Scott JK, Methods Enzymol. 217:228-257 (1993); O'Neil KT and Hoess RH, Curr. Opin. Struct. Biol. 5:443-449 (1995). Whether the VL domain or its derivative and the VH domain or its derivative can form a functional target binding site may also be evaluated by standard assays known in the art, for example, competition assays, enzyme-linked immunosorbant assay (ELISA), and radioimmunoassay (RIA). Likewise, the activity of the target binding site formed by association of two immunoglobulin-like domains may be determined using the above described methods. As used herein, a binding site is functional if it can bind to the desired target with an affinity of at least 10³ M⁻¹, preferably at least 10⁴ M⁻¹, more preferably at least 10⁵ M⁻¹, and most preferably at least 10⁶ M⁻¹

In another preferred embodiment, the first single chain Fv molecule and the first immunoglobulin-like domain are covalently linked via a first extra amino acid sequence, and the second single chain Fv molecule and the second immunoglobulin-like domain are covalently linked via a second extra amino acid sequence. Preferably, the first and second extra amino acid sequences associate with each other, so as to stabilize the association between the first and second polypeptides of the multivalent target binding protein. For example, the first and second extra amino acid sequences may be enriched with leucine residues in such a manner that they form a leucine zipper structure. More preferably, the first and second extra amino acid sequences covalently associate with each other. For example, the first and second extra amino acid sequences may be enriched with cysteine residues, so that they form disulfide bonds between each other.

In one embodiment, the first extra amino acid sequence comprises a light chain constant region domain (CL domain) or a derivative thereof, and the second extra amino acid sequence comprises a heavy chain constant region domain (CH domain) or a derivative thereof. Preferably, the CL domain or its derivative and the CH domain or its derivative associate with each other, so as to stabilize the association between the first and second polypeptide of the multivalent target binding protein. The CL domain or its derivative may associate with the CH domain or its derivative via non covalent interactions, such as hydrophobic interactions.

In a preferred embodiment, the first extra amino acid sequence comprises a kappa type light chain CL domain which has a cysteine corresponding to Cys214 according to Kabat's numbering, whereas the second extra amino acid sequence comprises a heavy chain hinge region, or a part thereof, which has a cysteine corresponding to Cys233 according to Kabat's numbering. The first and second extra amino acid sequences may be covalently linked via a disulfide bond between these two cysteine residues.

In another preferred embodiment, the first polypeptide of the multivalent target binding protein comprises a first scFv molecule covalently linked to an immunoglobulin light chain fragment which comprises the variable region VL and the constant region CL, and the second polypeptide of the multivalent target binding protein comprises a second scFv molecule covalently linked to an immunoglobulin heavy chain fragment which comprises the variable region VH and the constant region CH 1. *See* Figure 2. The VL region and VH region associate to form a target binding site. The CL region and CH1 region associate with each other to stabilize the multivalent target binding protein. Preferably, the first scFv molecule and the CL region are covalently linked via a first peptide linker which preferably consists of about 4 to about 15 amino acid residues. The second scFv molecule and the CH1 region are also preferably covalently linked via a second peptide linker which preferably consists of about 4 to about 15 amino acid residues. Preferably, the first peptide linker may have the amino acid sequence GGGS or EPKSADKTHTCPPCPGGGS, and the second peptide linker may have the amino acid sequence EPKSCGGGS or EPKSCDKTHTCPPCPGGGS. More preferably, the cysteine residue in the second peptide linker may form a disulfide bond with the CL region in a manner similar to the disulfide bond formed between an antibody light chain and heavy chain. The molecular weight of the multivalent target binding protein of this embodiment may be about 100 kDa.

In one embodiment, the first and second immunoglobulin-like domains may comprise humanized variable region domains. For instance, the complementarity-determining regions of a murine antibody may be grafted to the framework regions of a human antibody. See Sahagen et al. , J. Immunol., 137:1066-1074 (1986); Sun et al. , Proc. Natl. Acad. Sci. USA, 82:214-218 (1987); Nishimura et al., Cancer Res., 47:999-1005 (1987); Lie et al., Proc Natl. Acad. Sci. USA, 84:3439-3443 (1987); and US Patent No. 5,874,540. These references are incorporated herein by reference. Alternatively, human antibody variable regions may be used. Methods for isolating human antibodies are well known in the art, for example, by using a transgenic animal which has been modified to produce human antibodies, or from phage display of human antibody libraries. *See* US Patent Nos 6,075,181 and 5,969,108, which are hereby incorporated by reference. Whether a humanized variable region domain is able to associate with another variable region domain to form a functional target binding site may be determined using M13 bacteriophage display or other standard assays, for example competition assays, enzyme-linked immunosorbant assay (ELISA), and radioimmunoassay (RIA). The variable region domains of the first and second scFv molecules may likewise be humanized. Human antibody constant region domains may also be used to covalently link the first and second scFv molecules of a multivalent target binding protein to the first and second immunoglobulin-like domains, respectively.

In one embodiment, at least two of the three target binding sites of a multivalent binding protein may have different target binding specificities. For example, the first and second scFv molecules may have different amino acid sequences and possess different binding specificities. Each of the three target binding sites may have a different binding specificity from each other. As used herein, two binding sites have different target binding specificities if they do not have the same target binding specificity. Two binding sites have the "same" target binding specificity if they can bind to the same target with a similar binding affinity. Two target binding sites have a "similar" binding affinity if the ratio between their affinity constants for a given antigen or target is between about 0.2 to about 5. Two binding sites are identical if they have the same binding specificity to the same target.

In another embodiment, at least two of the three target binding sites of a multivalent binding protein may have the same target binding specificity. For example, the first and second scFv molecules may have the same amino acid sequence and possess the same binding specificity. The three target binding site may have the same target binding specificity. This may be achieved when the first immunoglobulin-like domain, the VL domain of the first scFv molecule and the VL domain of the second scFv molecule have the same amino acid sequence, and the second immunoglobulin-like domain, the VH domain of the first scFv molecule and the VH domain of the second scFv molecule also have an identical amino acid sequence. A target binding protein with at least two identical binding sites can exhibit an enhanced avidity to its target.

In yet another embodiment, the multivalent binding protein comprises at least two heterodimers, each heterodimer comprising a first and a second polypeptides. The first polypeptide comprises a first single chain Fv molecule and a first immunoglobulin-like domain which are covalently linked via a first extra amino acid sequence. The second polypeptide comprises a second single chain Fv molecule and a second immunoglobulin-like domain which are covalently linked via a second extra amino acid sequence. The first or second extra amino acid sequence of the first heterodimer may associate with the first or second extra amino acid sequence of the second heterodimer, preferably by covalent interactions, such as disulfide bonds.

As used herein, a molecule associates with another molecule if the two molecules have a propensity to join together. Association between two molecules may involve either covalently interactions or non-covalent interactions, or both covalent and non-covalent interactions. A molecule is linked or conjugated to another molecule if they associate with each other. As used herein, the terms "link" and "conjugate" are interchangeable.

### Peptide Linker Of Multivalent Target Binding Protein

The peptide linkers for the scFv molecules of the multivalent target binding protein preferably consist essentially of Gly and Ser residues. A preferred peptide linker is [GGGGS]₃. Glu and Lys residues may also be included. Suitable peptide linkers for a scFv molecule may be designed in accordance with the methods disclosed in US Patent No. 4,946,778.

The peptide linkers for the scFv molecules of the multivalent binding protein preferably comprise at least 12 amino acid residues. More preferably, the peptide linkers have at least 15 amino acid residues. Most preferably, the peptide linkers have about 15 to about 30 amino acid residues. A peptide linker shorter than 12 amino acids may reduce the flexibility between the VL and VH domains of a scFv molecule.

The first and second scFv molecules of a multivalent binding protein of the present invention may be either in the VL-L-VH configuration or in the VH-L-VL configuration. The two scFv molecules of the same multivalent binding protein may have the same or opposite configurations.

In one embodiment, the peptide linkers of the two scFv molecules of the multivalent binding protein comprise less than 12 amino acid residues, and the two scFv molecules have opposite configurations. For example, the first scFv molecule may have a VL-L-VH configuration, while the second scFv molecule has a VH-L-VL configuration. The two scFv molecules associate to form two target binding sites. In the above example, one target binding site may be formed via association between the VL domain of the first scFv molecule and the VH domain of the second scFv molecule, and the other target binding site may be formed by association between the VH domain of the first scFv molecule and the VL domain of the second scFv molecule. The binding specificity and affinity of thus formed two binding sites may be evaluated by standard assays known in the art, for example competition assays, enzyme-linked immunosorbant assay (ELISA), and radioimmunoassay (RIA).

In another embodiment, the first polypeptide of the multivalent target binding protein comprises a first scFv molecule covalently linked via a first peptide linker to an immunoglobulin light chain fragment, and the second polypeptide of the multivalent target binding protein comprises a second scFv molecule covalently linked via a second peptide linker to an immunoglobulin heavy chain fragment. The first and second peptide linkers may increase the flexibility of the scFv molecules with respect to other parts of the multivalent binding protein. This flexibility becomes significant when one target binding event hinders another target binding event due to , for example, the large size of the target. In a preferred embodiment, the immunoglobulin light chain fragment comprises the VL and CL regions, and the immunoglobulin heavy chain fragment comprises the VH and CH1 regions. The first and second peptide linkers preferably comprise at least 4 amino acid residues, more preferably at least 10 amino acid residues, and most preferably at least 15 amino acid residues. Preferably, the second peptide linker comprises a cysteine residue which is capable of forming a disulfide bond with the Cys 214 (Kabat's numbering) of the CL region of the immunoglobulin light chain fragment. For example, the second peptide linker may have the amino acid sequence EPKSCGGGS, and the first peptide linker may have the amino acid sequence GGGS. For another example, the second peptide linker may have the amino acid sequence EPKSCDKTHTCPPCPGGGS, and the first peptide linker may have the amino acid sequence EPKSADKTHTCPPCPGGGS.

### Conjugation Of Multivalent Target Binding Protein With An Agent

Additional amino acid residues may be added to either the N- or C-terminus of the first or the second polypeptide. The additional amino acid residues may comprise a peptide tag, a signal peptide, a cytokine, an enzyme (for example, a pro-drug activating enzyme), a peptide toxin such as pseudomonas extoxin, a peptide drug, a cytotoxic protein or other functional proteins. As used herein, a functional protein is a protein which has a biological function. A preferred functional protein is a cytotoxic protein. Adding extra amino acid residues at the N- or C-terminus of a protein is well known in the art. For instance, it may be achieved by ligating in-frame the DNA sequence encoding the additional amino acid residues with the DNA sequence encoding the first or second polypeptide. The ligation site may be at either the 5' or 3' end of the DNA sequence encoding the first or second polypeptide. The additional amino acid residues preferably does not significantly affect the binding specificity or affinity of the multivalent binding protein. A target binding protein's binding specificity is significantly affected if the modified protein binds to its purported target at an affinity of less than 10³ M⁻¹. A target binding protein's binding affinity is significantly affected if the binding affinity of the modified protein to its purported target is 10 times less than that of the unmodified protein.

In one embodiment, drugs, toxins, radioactive compounds, enzymes, cytotoxic proteins, chelates, cytokines and other functional agents may be conjugated to the multivalent target binding protein, preferably through covalent attachments to the side chains of the amino acid residues of the multivalent target binding protein, for example amine, carboxyl, phenyl, thiol or hydroxyl groups. Various conventional linkers may be used for this purpose, for example, diisocyanates, diisothiocyanates, bis(hydroxysuccinimide) esters, carbodiimides, maleimide-hydroxysuccinimide esters, glutaraldehyde and the like. Conjugation of agents to the multivalent protein preferably does not significantly affect the protein's binding specificity or affinity to its target. As used herein, a functional agent is an agent which has a biological function. A preferred functional agent is a cytotoxic agent.

In another embodiment, cytotoxic agents may be conjugated to a polymeric carrier, and the polymeric carrier may subsequently be conjugated to the multivalent target binding protein. For this method, see Ryser et al., Proc. Natl. Acad. Sci. USA, 75:3867-3870, 1978, US Patent No. 4,699,784 and US Patent No. 4,046,722. Conjugation preferably does not significantly affect the binding specificity or affinity of the multivalent binding protein.

Many drugs and toxins are known to have cytotoxic effects on tumor cells or microorganisms. These drugs and toxins may be found in compendia of drugs and toxins, such as the Merck Index or the like.

In one embodiment, at least one N-glycosylation sequence may be introduced into either the first or second polypeptide of the multivalent target binding protein. See Hansen et al., U.S. Patent No. 5,443,953, and Leung et al., U.S. Provisional Patent Application 60/013,709, where a N-glycosylation sequence is introduced to the VL (HCN1 site) or CH1 (HCN5 site) region of an antibody. Preferably, the glycosylation sequence may be inserted at a site distant from the target binding site, such that glycosylation of the sequence does not significantly affect the binding specificity or affinity of the multivalent target binding protein. More preferably, the glycosylation site may be inserted at least 4.1Å away from the target binding site. Most preferably, the N-glycosylation site may be introduced outside the first and second immunoglobulin-like domains and the first and second scFv molecules. In a preferred embodiment, a N-glycosylation site may be engineered within the first and second extra amino acid sequences, such as an immunoglobulin constant region domain which covalently links the first or second inununoglobulin-like domain to the first or second scFv molecule, respectively. Computer modeling may help locate suitable sites for introducing the N-glycosylation recognition sequence.

N-glycosylation recognition sites may be engineered into the first or second polypeptide using site-directed mutagenesis. Whenever possible, the mutations introduced are conservative in nature, so as to maintain the final tertiary structure of the protein domains. A conservative mutation generally involves substitution of one for another by similar size and clinical properties. For example, a preferred N-glycosylation recognition sequence is NXT or NXS, wherein N denotes asparagine, T denotes threonine, S denotes serine and X denotes any amino acid residue. Replacement of a glutamine (Q) residue with an asparagine (N) residue would be considered a conservative substitution. Possible perturbations in the final tertiary structure may be minimized by carefully choosing sequences that only one single amino acid substitution therein is sufficient to provide a potential glycosylation site.

Insertion of the N-glycosylation sequence is described only as an example. The principles involved are equally applicable to O-glycosylation. O-glycosylation is known to occur at either threonine or serine. The acceptor sequence for O-linked glycosylation is relatively ill defined. *See* Wilson et al., Biochem. J., 275: 526 (1991). There could be a bias for higher content of proline, serine and threonine in these regions, but accessibility, rather than the exact primary sequence may determine whether a particular threonine or serine residue will be O-glycosylated. Nevertheless, potential O-glycosylation sequences, such as those identified in other antibodies known to have O-glycosylation can be used as the standard sequences for grafting into different positions in the target binding proteins of interest. *See* Chandrashekarkan et al., J. Biol. Chem., 259: 1549 (1981), Smyth and Utsumi, Nature, 216: 322 (1967), Kim et al., J. Biol. Chem., 269: 12345 (1994). Insertion of glycosylation recognition sequences, glycosylation of the introduced sequences, or any other modifications preferably do not significantly affect the binding specificity and affinity of the multivalent target binding protein.

In another embodiment, a carbohydrate chain may be covalently linked to an engineered glycosylation sequence. Covalent attachment of a carbohydrate chain may be achieved by expressing the multivalent binding protein which comprises the glycosylation recognition sequence in a eukaryotic cell. A signal peptide sequence may preferably be introduced at the N-terminus of the first or second polypeptide of the multivalent binding protein. When expressed in a eukaryotic cell, the first or second polypeptide with the signal peptide may translocate from cytosol to endoplasmic reticulum (ER), where the polypeptide can be glycosylated via the engineered glycosylation recognition sequence.

In yet another embodiment, enzymes, toxins, cytokines, drugs, chelates, cytotoxic proteins, radioactive compounds or other cytotoxic agents may be attached to the carbohydrate chain which has been incorporated into the multivalent binding protein via the engineered glycosylation recognition site. To conjugate an agent to a carbohydrate chain, the hemiacetal rings in the carbohydrate chain may be chenically oxidized to generate reactive aldehyde groups. Aldehyde groups thus formed may be covalently bonded to the amino groups of a protein or an agent through Schiff bases. For general methods of attaching proteins or agents to a carbohydrate chain, see Hansen et al., U.S. Patent No. 5,443,953, and Leung et al., U.S. Provisional Patent Application 60/013,709.

### Construction of Expression Vectors for Multivalent Target Binding Protein

The expression vectors for the first or second polypeptides of the multivalent binding protein may be obtained by in-frame ligation of the DNA sequences encoding the immunoglobulin-like domain, the scFv molecule or the extra amino acid sequence using DNA ligation techniques as appreciated by one of skill in the art. See Sambrook et al., Molecular Cloning: A Laboratory Manual, Cold Spring Harbor Laboratory Press, 2nd Ed. (1989). A peptide linker which covalently connects the scFv molecules to the other parts of the multivalent binding protein may be introduced by PCR techniques, for example, using a primer which incorporates the DNA sequence encoding the peptide linker.

The DNA sequences encoding the variable and constant region domains of an antibody may be obtained from published sources or can be obtained by standard procedures known in the art. For example, Kabat et al. , Sequences of Proteins of Immunological Interest, 4th ed (1991), published by The U.S. Department of Health and Human Services, discloses sequences of most of the antibody variable regions that have been described prior to its publication date.

General techniques for the synthesis of antibody variable or constant regions are described, for example, by Orlandi et al., Proc. Nat'l Acad. Sci. USA, 86:3833 (1989) and Larrick et al., Methods: A Companion to Methods in Enzymology, 2:106 (1991). Also see, Ward et al., "Genetic Manipulation and Expression of Antibodies," in MONOCLONAL ANTIBODIES: PRINCIPLES AND APPLICATIONS, pages 137-185 (Wiley-Liss, Inc. 1995), and Courtenay-Luck, "Genetic Manipulation of Monoclonal Antibodies," in MONOCLONAL ANTIBODIES: PRODUCTION, ENGINEERING AND CLINICAL APPLICATION, Ritter et al. (eds.), pages 166-179 (Cambridge University Press 1995).

DNA sequences for the variable and constant regions of an antibody may also be obtained through reverse transcription of the mRNAs which encode the antibody. The source of mRNAs for antibodies may be obtained from a wide range of hybridomas. See, for example, the catalogue of ATCC Cell Lines and Hybridomas, American Type Culture Collection, 20309 Parklawn Drive, Rockville Md., USA (1990). Hybridomas secreting monoclonal antibodies reactive with a wide variety of antigens are listed therein, and are available from the collection. These cell lines or others of similar nature may be utilized as a source of mRNAs coding for the variable and constant regions of antibodies.

Variable and constant regions of antibodies may also be derived by immunizing an appropriate vertebrate, normally a domestic animal, and most conveniently a mouse. The immunogen will be the antigen of interest, or where a hapten is of interest, an antigenic conjugate of the hapten to an antigen such as keyhole limpet hemocyanin (KLH). The immunization may be carried out conventionally with one or more repeated injections of the immunogen into the host mammal, normally at two to three week intervals. Usually three days after the last challenge, the spleen is removed and dissociated into single cells to be used for cell fusion to provide hybridomas from which mRNAs can readily be obtained by standard procedures known in the art. DNA sequences may be obtained through reverse transcription of the mRNAs. The above procedures can produce an antibody which may be specific to any selected immunogenic antigens, for example, a cell surface protein, a T cell marker such as CD 28 and CD3, a Fc receptor, a drug, a toxin, a cytokine, an enzyme, a cytotoxic protein, a chelate, a tumor antigen, or a chemical compound which may be radioactive. The DNA sequences coding for the variable or constant regions of the antibody may be used to construct the multivalent target binding protein of the present invention.

Variable and constant regions of antibodies may be obtained using M13 bacteriophage display. See Burton et al, Adv. Immuno. 57:191-280 (1994). Essentially, a cDNA library for antibodies is generated from mRNAs obtained from a population of antibody-producing cells, such as B-lymphocytes. Amplified cDNAs are cloned into M13 phage display vectors creating a library of phage which express the antibody fragments on the phage surface. Phage which displays the antibody fragment of interest is selected using the affinity to the antigen. The selected phage is amplified to produce the antibody of interest.

Construction of the DNA sequences for scFv molecules is disclosed, for example, in European Patent Application No. 239400 and U.S. Patent No. 4,946.778. Construction of scFv sequences also is described in R.E. Bird and B.W. Walker, "Single Chain Antibody Variable Regions," TIBTECH, Vol 9: 132-137 (1991). In addition, the DNA sequences encoding the VL and VH regions of scFv molecules may be obtained from antibodies which can be prepared as described above.

A signal peptide, preferably with antibody gene origin, may be added to the N-terminal end of a target binding protein by routine DNA cloning techniques, for instance, by a PCR using a 5' end primer comprising the signal peptide sequence. Alternatively, a signal peptide may be incorporated through reverse transcription of an antibody mRNAs. The mRNAs encoding a naturally-occurring antibody usually comprises signal peptide sequences. Reverse transcription of the mRNAs will produce a DNA sequence which may encode both a signal peptide and an antibody variable region. The DNA sequence thus obtained may be used to construct the first or second immunoglobulin-like domain of the multivalent target binding protein.

DNA sequence may be determined by methodologies described in Sanger et al., Proc. Natl. Acad. Sci., USA, 74: 5463 (1977).

### Expression of Multivalent Target Binding Protein

Methods for introducing a DNA vector into a host cell are well known in the art. These methods include, but are not limited to, electroporation, calcium phosphate, cationic lipid, gene gun, and Biolistic (Bio-Rad) method.

To express the first and second polypeptides of a multivalent target binding protein, the DNA sequences encoding the two polypeptides must be operably linked to regulatory sequences controlling transcriptional and translational expressions in host cells. Regulatory sequences that control transcription include promoters and enhancers. The host cell may be either prokaryotic or eukaryotic. The expression vectors may also include a marker gene for selection of host cells that carry the expression vectors.

Suitable promoters for expression in a prokaryotic host can be repressible, constitutive, or inducible. These promoters are well-known to those skilled in the art. These promoters include, but are not limited to, promoters capable of recognizing the T4, T3, Sp6 and T7 polymerases, the P_{R} and P_{L} promoters of bacteriophage lambda, the trp, recA, heat shock, and lacZ promoters of *E. coli*, the α-amylase and the σ²⁸-specific promoters of *B. subtilis*, the promoters of the bacteriophages of *Bacillus*, *Streptomyces* promoters, the *int* promoter of bacteriophage lambda, the *bla* promoter of the β-lactamase gene of pBR322, and the CAT promoter of the chloramphenicol acetyl transferase gene. Prokaryotic promoters are reviewed by Glick, J. Ind. Microbiol., 1: 277 (1987) and Watson et al., MOLECULAR BIOLOGY OF THE GENE, 4th Ed., Benjamin Cummins (1987).

A preferred prokaryotic host is *E. coli*. Preferred strains of *E. coli* include Y1088, Y1089, CSH18, ER1451, and ER1647. See, for example, Brown (Ed.), MOLECULAR BIOLOGY LABFAX, Academic Press (1991). An alternative preferred host is *Bacillus subtilus*, including such strains as BR151, YB886, MI119, MI120, and B170. See, for example, Hardy, "Bacillus Cloning Methods," in DNA CLONING: A PRACTICAL APPROACH, Glover (Ed.), IRL Press (1985).

Methods for expressing antibodies in prokaryotic hosts are well-known to those skilled in the art. See, for example, Ward et al., "Genetic Manipulation and Expression of Antibodies," in MONOCLONAL ANTIBODIES: PRINCIPLES AND APPLICATIONS, pages 137-185 (Wiley-Liss, Inc. 1995). Moreover, expression systems for cloning antibodies in prokaryotic cells are commercially available. For example, the IMMUNO ZAP^{™} Cloning and Expression System (Stratagene Cloning Systems; La Jolla, CA) provides vectors for the expression of antibody light and heavy chains in *E. coli*. One skilled in the art would understand that the techniques for expressing and cloning antibodies in prokaryotic cells may be employed for expressing and cloning the multivalent binding protein of the present invention without undue experimentation.

Alternatively, the first and second polypeptides of the multivalent binding protein of the present invention may be expressed in eukaryotic host cells. Eukaryotic host cells include mammalian, insect and yeast cells. Preferably, the eukaryotic host cell is a mammalian cell. Mammalian cells may provide proper post-translational modifications to the expressed polypeptides. Suitable mammalian host cells may include COS-7 cells (ATCC CRL 1651), non-secreting myeloma cells (SP2/0-AG14; ATCC CRL 1581), rat pituitary cells (GH₁; ATCC CCL 82), and rat hepatoma cells (H-4-II-E; ATCC CRL 1548).

For a mammalian host, the transcriptional and translational regulatory signals may be derived from viral sources, such as adenovirus, bovine papilloma virus, and simian virus. In addition, promoters from mammalian expression products, such as actin, collagen, or myosin, may be employed. Preferably, a metallothionine promoter may be used. Alternatively, a prokaryotic promoter, such as the bacteriophage T3 RNA polymerase promoter, may be employed, wherein the prokaryotic promoter is regulated by a eukaryotic promoter, for example, *see* Zhou et al., Mol. Cell. Biol., 10:4529 (1990), and Kaufman et al., Nucl. Acids Res., 19:4485 (1991). Transcriptional initiation regulatory signals may be selected so that expression of the genes can be modulated, for example, be able to subject to repression or activation.

In general, eukaryotic regulatory regions include a promoter region sufficient to direct the initiation of RNA synthesis. Such eukaryotic promoters include the promoter of the mouse metallothionein I gene (Hamer et al., J. Mol. Appl. Gen. 1:273 (1982)); the TK promoter of Herpes virus (McKnight, Cell 31:355 (1982)); the SV40 early promoter (Benoist et al., Nature (London) 290:304 (1981)); the Rous sarcoma virus promoter; the cytomegalovirus promoter (Foecking et al., Gene 45:101 (1980)); the yeast *gal4* gene promoter (Johnston, et al., Proc. Natl. Acad. Sci. (USA) 79:6971 (1982); Silver, et al., Proc. Natl. Acad. Sci. (USA) 81:5951 (1984)); and the IgG promoter (Orlandi et al., Proc. Natl. Acad. Sci. USA 86:3833 (1989)).

Strong regulatory sequences are the preferred regulatory sequences of the present invention. Examples of such preferred regulatory sequences include the SV40 promoter-enhancer (Gorman, "High Efficiency Gene Transfer into Mammalian cells," in DNA CLONING: A PRACTICAL APPROACH, Volume II, Glover (Ed.), IRL Press pp. 143-190 (1985)), the hCMV-MIE promoter-enhancer (Bebbington et al., BiolTechnology 10:169 (1992)), and antibody heavy chain promoter (Orlandi et al., Proc. Natl. Acad. Sci. USA 86:3833 (1989)). Also preferred are the kappa chain enhancer for the expression of the light chain and the IgH enhancer (Gillies, "Design of Expression Vectors and Mammalian Cell Systems Suitable for Engineered Antibodies," in ANTIBODY ENGINEERING: A PRACTICAL GUIDE, C. Borrebaeck (Ed.), W.H. Freeman and Company, pp. 139-157 (1992)).

The DNA sequence encoding the first or second polypeptide, which is operably linked to a promoter, may be introduced into eukaryotic host cells as a non-replicating DNA molecule. These DNA sequences may be either in a linear form or, more preferably, in a closed covalent circular form. Because these DNA molecules are incapable of autonomous replication, the expression of the encoded proteins may occur through the transient expression of the introduced DNA sequences. Preferably, permanent expression may be used, which may occur when the introduced DNA sequences are integrated into the host chromosome.

Preferably, the introduced DNA sequence will be incorporated into a plasmid or viral vector that is capable of autonomous replication in the recipient host. Several possible vector systems are available for this purpose. One class of vectors utilize DNA elements which provide autonomously replicating extra-chromosomal plasmids, derived from animal viruses such as bovine papilloma virus, polyoma virus, adenovirus, or SV40 virus. A second class of vectors relies upon the integration of the desired genomic or cDNA sequences into the host chromosome. Additional elements may also be needed for optimal synthesis of mRNA. These elements may include splice signals, as well as transcription promoters, enhancers, and termination signals. The cDNA expression vectors incorporating such elements include those described by Okayama, Mol. Cell. Biol. 3:280 (1983), Sambrook et al., Molecular Clowning: A Laboratory Manual, Cold Spring Harbor Laboratory Press, New York, 2nd Ed. (1989), and Fouser et al., BiolTechnology 10:1121 (1992). Genomic DNA expression vectors which include intron sequences may also be used. See generally, Lerner et al. (Eds.), NEW TECHNIQUES IN ANTIBODY GENERATION, Methods 2(2) (1991).

Additionally, it is preferred that the expression vector contains a selectable marker, such as a drug resistance marker or other marker which causes expression of a selectable trait by the host cell. "Host cell" refers to cells which can be recombinantly transformed or transfected with vectors constructed using recombinant DNA techniques. A drug resistance or other selectable marker is intended in part to facilitate in the selection of transformed or transfected host cells. For example, G418 can be used to select transfected cells carrying an expression vector having the aminoglycoside phosphotransferase gene. *See* Southern et al., J. Mol. Appl. Gen., 1:327 (1982). Alternatively, hygromycin-B can be used to select transfected host cells carrying an expression vector having the hygromycin-B-phosphotransferase gene. *See* Palmer et al., Proc. Natl. Acad. Sci. USA, 84:1055 (1987). Alternatively, aminopterin and mycophenolic acid can be used to select transfected cells carrying an expression vector having the xanthine-guanine phosphoribosyltransferase gene. *See* Mulligan et al., Proc. Natl. Acad. Sci. USA, 78:2072 (1981). Preferably, methotrexate can be used to select transfected cells, such as transfected SP2/0 cells, which carry an expression vector having the DHFR gene, and the selected transfected cells may subsequently be subject to step-wise increases in the concentration of methotrexate, in order to increase the production of the desired protein. For a host cell which carries two expression vectors simultaneously, each vector comprising the DNA sequence encoding a different polypeptide of the multivalent binding protein, it is preferred that each vector is designed to have a different selectable marker so that the host cell may be selected by using a combination of two drugs.

Additionally, the presence of a selectable marker, such as a drug resistance marker, may be of use in keeping contaminating microorganisms from multiplying in the culture medium. In this embodiment, such a pure culture of transformed or transfected host cells may be obtained by culturing the cells under conditions which require for survival the phenotype associated with the selectable marker.

It is preferred that the expression vectors and the inserts which code for the first or second polypeptides of the multivalent binding protein of the present invention have compatible restriction sites at the insertion junctions and that those restriction sites are unique to the areas of insertion. Both vector and insert are treated with restriction endonucleases and then ligated by any of a variety of methods such as those described in Sambrook et al., Molecular Cloning: A Laboratory Manual, Cold Spring Harbor Laboratory Press, New York, 2nd Ed. (1989).

In one embodiment, the expression vector comprising the DNA sequence encoding the first polypeptide of the multivalent target binding protein also comprises the DNA sequence encoding the second polypeptide of the protein. Each of these DNA sequences is operably linked to a separate set of regulatory sequences controlling transcriptional and translational expressions. The expression vector may be introduced into either prokaryotic or eukaryotic host cells and expressed therein. The multivalent target binding protein preferably may be assembled within the host cells, and isolated according to the methods described below. Alternatively, the expressed polypeptides may be isolated, and then associate to form the multivalent binding protein *in vitro*.

In another embodiment, the DNA sequences encoding the first and second polypeptides of the target binding protein may be cloned into different expression vectors. Each DNA sequence is operably linked to regulatory sequences controlling transcriptional and translational expressions. Each vector is introduced into either prokaryotic or eukaryotic host cells and expressed therein. The produced first and second polypeptides are isolated or concentrated according to the methods described below. Then the isolated or concentrated first and second polypeptides are mixed together to associate to form the multivalent binding protein. The final product of the multivalent binding protein may be isolated using the methods as described below. Alternatively, the two vectors encoding the first and second polypeptides may be introduced into the same host cell for co-expression. The expressed first and second polypeptides may be assembled within the host cell, and then isolated accordingly.

### Isolation of Multivalent Target Binding Protein

The transfected or transformed host cells may be selected and cultured, and then lysed using detergents or osmotic shocking. For an expression construct having a signal peptide which allows the expressed protein to be secreted, the supernant of the cell culture, together with the cell lysate, may be retained for isolation of the expressed protein. The expressed protein may be isolated or concentrated using standard techniques known in the art, such as affinity chromatography, protein G affinity chromatography, gel filtration chromatography, and ion-exchange chromatography. See Coligan et al. (eds.), CURRENT PROTOCOLS IN IMMUNOLOGY, John Wiley & Sons (1991), for detailed protocols. The affinity chromatography column may be coupled with a target which binds to at least one of the three target binding sites.

Polypeptides expressed in prokaryotic host cells may be concentrated in refractile bodies or inclusion bodies. Inclusion bodies may be purified by lysing the cells, and repeatedly centrifuging the lysed cells and washing the resultant pellets. The final pellets contain isolated inclusion bodies. The isolated inclusion bodies may be solubilized using guanidine-HCI, followed by gel filtration chromatography, to isolate the expressed protein. Guanidine-HCl treatment is especially suitable for the multivalent target binding protein which has its first and second polypeptides covalently linked via at least a disulfide bond.

Affinity chromatography is well known to one of skill in the art. Briefly, the purported target of the multivalent target binding protein may be coupled to the matrix of the chromatography column, such as agarose beads. The expressed multivalent target binding protein or one of its two polypeptide may be retained by the target-coupled affinity column if they possess a functional binding site specific to the target. The retained proteins may subsequently be eluted. Protein G affinity column may also be used to purify the multivalent target binding protein, as will be appreciated by one of ordinary skill in the art.

Ion-exchange chromatography is well-known to those of ordinary skill in the art. Most protein has either positive or negative charges. Thus, a chromatography column with the opposite type of charges may retain the proteins.

Gel filtration chromatography uses a gel-like material to separate proteins on the basis of their molecular weights. A "gel" is usually a matrix of water and a polymer, such as agarose or polymerized acrylamide. The present invention encompasses the use of gel filtration HPLC (high performance liquid chromatography), as will be appreciated by one of ordinary skill in the art.

Standard recovery and collection procedures are well known in the art. Recovering the expressed polypeptide or multivalent target binding protein preferably comprises collecting eluate fractions which contain the peak of interest from either an affinity column, an ion exchange column or a gel filtration column. Manual and automated fraction collection are well-known in the art. Subsequent processing may involve lyophilization of the collected eluate to produce a stable solid, or further purification.

The activity, including binding specificity and affinity, of the isolated polypeptide or multivalent binding protein may be assessed by standard assays known in the art, for example competition assays, enzyme-linked immunosorbant assay (ELISA), and radioimmunoassay (RIA).

### Stabilization of Multivalent Target Binding Protein

In one embodiment, the first and second polypeptides of the multivalent target binding protein may be stabilized via covalent interactions, such as disulfide bonds. For instance, Each of the first and second polypeptides may comprise a cysteine-rich extra amino acid sequence. These extra amino acid sequences may form disulfide bonds between each other, and therefore stabilize the association between the first and second polypeptides. In a preferred embodiment, the first polypeptide may comprise a CL region which covalently links the first scFv molecule to the first immunoglobulin-like domain, and the second polypeptide may comprise a complete or partial heavy chain hinge region. The CL region may be covalently linked to the hinge region via a disulfide bond.

Formation of disulfide bonds may occur during the synthesis of the multivalent target binding protein in host cells. Suitable host cells may include prokaryotic cells (such as *E. Coli*), yeast, and insect cells. Preferred host cells include cultured mammalian cells. Formation of disulfide bonds may also be driven *in vitro* by oxidation using the method as described in Kostelny et al., J. Immunol., 148:1547-1553 (1992). Under this method, the first and second polypeptides of the multivalent binding protein are mixed and dialyzed against a redox buffer. The final product may be purified by either gel filtration chromatography or affinity chromatography, in which the affinity chromatography column is coupled with the target which binds to the target binding site formed by the association between the first and second immunoglobulin-like domains.

To prevent undesirable disulfide bonds, undesirable cysteine residues may be replaced by non-cysteine residues, for example, by site-directed mutagenesis. Whenever possible, the mutations introduced are conservative in nature, so as to maintain the final tertiary structure of the protein domains. For example, a substitution of cysteine for serine may be considered a conservative substitution under certain conditions. Substitution of cysteine residues preferably does not significantly affect the binding specificity or stability of the multivalent target binding protein.

Where the first polypeptide comprises a light chain fragment comprising the VL and CL regions and the second polypeptide comprises a heavy chain fragment comprising the VH and CH1 region, see Figure 2, the amino acid residues involved in the interactions between the light chain fragment and the heavy chain fragment may be subject to mutagenesis in order to enhance the association between the two fragments. Suitable amino acid residues for mutagenesis may be determined based on the crystal structure of an antibody. The crystal structure of an antibody is known in the art. Candidate mutagenesis may be directed to introducing ionic bonds or disulfide bonds, or increasing hydrophobic interactions or the number of hydrogen bonds. Mutation of these residues preferably does not significantly change the binding specificity or affinity of the multivalent binding protein. The final product of mutagenesis may be isolated using affinity chromatography which is coupled with the purported target. If the mutagenesis does not significantly affect the binding activity of the multivalent binding protein, the protein may be retained by the affinity column and recovered using routine collection techniques.

### Application of Multivalent Target Binding Protein

The multivalent target binding protein of the present invention has many applications. Essentially all of the known uses for which monoclonal or polyclonal antibodies, or fragments thereof, can be addressed by the multivalent target binding protein of the present invention. A multivalent binding protein may be detectably-labeled. Types of labels are well-known to those of ordinary skill in the art. They include radiolabeling, chemiluminescent labeling, fluorochromic labeling, and chromophoric labeling. Other uses include imaging the internal structure of an animal (including a human) by administering an effective amount of a labeled form of the multivalent protein and measuring detectable radiation associated with the animal. They also include improved immunoassays, including sandwich immunoassay, competitive immunoassay, and other immunoassays wherein the labeled antibody can be replaced by the labeled multivalent target binding protein of the present invention.

The multivalent target binding protein may be used to recruit cytotoxic cells, such as natural killer (NK) or cytotoxic T cells, to specific cellular targets, such as tumor cells or infectious cells. See Staerz et al., Nature, 314:628 (1985), and Songilvilai and Lachmann, Clin. Exp. Immunol., 79:315 (1990). The multivalent target binding protein may also be used to deliver toxins, drugs, chelates, cytokines, enzymes such as pro-drug activating enzymes, radioactive compounds, cytotoxic proteins or other cytotoxic agents to tumor cells or infectious cells. The use of multivalent targeting binding proteins which are conjugated, either covalently or non-covalently, with radioactive compounds or other cytotoxic agents offers the possibility of delivering these agents directly to the tumor or lesion sites, thereby limiting the exposure of normal tissues to toxic agents. *See* Goldenberg, Semin. Nucl. Med., 19: 332 (1989). In recent years, multivalent target binding protein (including antibodies) based therapy and its accuracy in the localization of tumor-associated antigens have been successfully demonstrated both in the laboratory and clinical studies. See, e.g., Thorpe, TIBTECH, 11: 42 (1993); Goldenberg, Scientific American, Science & Medicine, 1: 64 (1994); US Patent Nos. 4,925,922 and 4,916,213; US Patent No. 4918163; US Patent No. 5,204,095; US Patent No. 5,196,337; and US Patent Nos. 5,134,075 and 5,171,665. In addition, multivalent target binding proteins may be useful for targeting tumor cells or infectious cells in *in vitro* conditions, for example, treating or detecting tumor cells or infectious cells in isolated biological samples. The multivalent target binding protein may also be useful for *ex vivo* purging of leukemia cells from bone marrow. *See* Kaneko et al., Blood, 81:1333-1341 (1993).

In one embodiment, the multivalent binding protein has at least one target binding site capable of binding to either a cytotoxic agent or a cytotoxic cell, and has at least another binding sites, preferably two other binding sites, capable of binding to antigens on tumor cells or infectious cells.

In another embodiment, the multivalent binding protein has at least one binding site, preferably three binding sites, capable of binding to antigens on tumor cells or infectious cells. Cytotoxic agents are conjugated to the multivalent binding protein, preferably by covalent attachments such as via the side chains of the amino acid residues of the protein, or via the carbohydrate chain engineered to the protein.

The multivalent target binding protein may be used for detecting or treating tumor cells, infectious cells, tumors or infectious lesions. Preferably, the multivalent target binding protein may be directly applied to a human patient or a non-human animal to treat a particular tumor or infectious lesion or to determine whether the subject has a particular tumor or infectious lesion. *See* Doussal et al., Int. J. Cancer, Supplement 7:58-62 (1992); Peltier et al.,. J. Nucl. Med., 34: 1267-1273 (1993); Somasundaram et al.. Cancer Immunol. Immumother., 36: 337-345 (1993); Bruynck et al., Br. J. Cancer, 67: 436-440 (1993). For example, a multivalent target binding protein may have a tumor-antigen binding site and a hapten binding site. This protein may be introduced into a patient via injection, and the injected protein binds to the tumor antigen at the tumor site *in vivo*. A radioactively labeled hapten, such as a metal chelate, is then introduced to the patient via injection, and localized to the tumor site by binding to the protein via the happen binding site, thereby enabling detection or treatment of the tumor. In the above example, the radioactively labeled hapten may also be conjugated to the multivalent target binding protein, for example, via the carbohydrate chain engineered to the multivalent target binding protein.

In another embodiment, the multivalent target binding protein may have one target binding site specific to a target cell, and two target binding sites specific to cell-surface antigens of effector cells. Preferred target cells include tumor cells, infectious cells, or any other types of undesirable cells. Effector cells are those cells that can generate or participate in generation of a physiological response, such as an immune response, against an antigen or a target cell. Preferred effector cells include, but are not limited to, T cells, NK cells and macrophage cells. The two target binding sites specific to effector cells may bind to the same or different surface antigens on the effector cells. For example, the two target binding sites may bind to a surface antigen of cytotoxic T cell and a surface antigen of NK cell, respectively. Such a multivalent target binding protein is capable of recruiting both cytotoxic T cells and NK cells to a single target cell, and therefore may create an enhanced immune response against the target cell.

In a preferred embodiment, the multivalent target binding protein may have one target binding site specific to a target cell (e.g. a tumor cell), and two target binding sites specific to two different surface antigens on a single effector cell (e.g. a T cell). Thus, the multivalent target binding protein may bind to a single effector cell via two different surface antigens, which may trigger two different signal transduction pathways in the effector cell. Activation of two signal pathways in a single effector cell may produce an enhanced physiological response from the effector cell.

In another preferred embodiment, the multivalent target binding protein has one target binding site capable of binding to a tumor antigen or an antigen on an infectious cell, and the other two target binding sites capable of binding to T cell surface proteins CD3 and CD28, respectively. This multivalent target binding protein is, therefore, able to trigger two different signal transduction pathways in the T cell, one via CD3 and the other via CD28, so as to create an enhanced immune response against the targeted tumor or infectious cell. *See* Holliger et al., Cancer Research, 59:2099 (1999).

In another embodiment, one of the three target binding site of the multivalent target binding protein binds to a cytotoxic agent or a surface antigen of an effector cell (such as CD8 or CD4 of T cell). The other two target binding sites bind to tumor antigens, such as CEA(anti-carcinoembryonic antigen) or CSAp (Colon-Specific Antigen p). Both CEA and CSAp are found to be expressed on the surface of colon cancers. With two target binding sites binding to the same tumor antigen, the multivalent target binding protein may have a higher avidity to the targeted tumor cells, thus limiting the exposure of normal tissues to the cytotoxic affects associated with the cytotoxic agent or effector cell. In yet another embodiment, the two tumor-antigen binding sites may have different binding specificities, for example, one binding to CEA and the other binding to CSAp. Having two different tumor-target binding specificities may increase the chance of tumor targeting and therefore reduce the chance of tumor evasion resulting from antigen modulation..

In another embodiment, either the first or second polypeptide of the multivalent target binding protein is covalently linked to additional amino acid residues at either N- or C-terminus thereof. These additional amino acid residues may comprise a peptide tag, a signal peptide, an enzyme such as a pro-drug activating enzyme, a cytokine, a peptide toxin, a peptide drug, a cytotoxic protein or other functional proteins. This multivalent target binding protein may be useful for treating or diagnosing tumors. For example, a peptide toxin, such as pseudomonas exotoxin, or a cytokine, such as IL-1, IL-2, IFN.gamma., TNF.alpha. and GM-SF, may be added at the N- or C-terminus of the multivalent target binding protein, which preferably has three tumor-antigen binding sites. With three tumor-antigen binding sites, the multivalent binding protein may have a higher avidity to the tumor, and therefore may more effectively deliver the attached toxin or cytokine to the targeted tumor site. For another example, the multivalent target binding protein which has three tumor-antigen binding sites may be attached with a peptide tag which can be recognized by another radiolabeled antibody. This multivalent binding protein may be useful for detecting or treating tumors *in vivo*. With three tumor binding sites, this multivalent target binding protein may provide a more sensitive way for detection and treatment of tumors.

In a preferred embodiment, the multivalent target binding protein may be employed for pretargeting using the "affinity enhancement system." For example, the multivalent target binding protein may have three target binding sites, two for tumor antigens and one for a hapten such as the In-DTPA hapten. The two tumor antigen binding sites preferably bind to the same antigen, or different antigens associated with the same tumor cell. A subject, which may be, for example, a human or a non-human animal, may be pretreated with the target binding protein. As used herein the terms subject and patients can be used interchangeably. At a predetermined time, the unbound target binding proteins are cleared from the subject. The subject is then administered with a peptide carrier carrying the hapten, preferably the peptide carrier carrying at least two haptens. The peptide carrier may be radiolabeled, or conjugated with drugs, toxins or other toxic agents, and therefore may exert a inhibitory effect on the growth of the targeted tumor cells.

The multivalent target binding protein of the present invention may be formulated according to known methods to prepare pharmaceutically useful compositions or medicaments, whereby the protein is combined in a mixture with a pharmaceutically acceptable carrier. Sterile phosphate-buffered saline is one example of a pharmaceutically acceptable carrier. Other suitable carriers are well-known to those skilled in the art. See, for example, REMINGTON'S PHARMACEUTICAL SCIENCES, 19th Ed. (Mack Publishing Co. 1995), and GILMAN'S THE PHARMACOLOGICAL BASIS OF THERAPEUTICS, 7th Ed. (MacMillan Publishing Co. 1985).

Administration of a multivalent target binding protein to a patient or a non-human animal may be intravenous, intraarterial, intraperitoneal, intramuscular, subcutaneous, intrapleural, intrathecal, by perfusion through a regional catheter, or by direct intralesional injection. When administering a multivalent target binding protein by injection, the administration may be by continuous infusion or by single or multiple boluses.

For treating or detecting tumors or infectious lesions, or for eliciting an immune response against tumors or infectious lesions, the multivalent target binding protein or another agent is administered to a patient or a non-human animal in an effective amount. For purpose of treating tumors or infectious lesions, a multivalent target binding protein or another agent is administered in an "effective amount" if the amount administered is physiologically significant. An amount is physiologically significant if it results in a detectable change in the physiology of at least one targeted cell in the recipient patient or non-human animal, preferably if it results in a detectable change in the physiology of the targeted tumor or infectious lesion in the recipient patient or non-human animal. In particular, an amount is physiologically significant for treating a tumor or an infectious lesion if it results in an inhibitory effect on the growth of at least one targeted tumor or infectious cell, preferably if it results in an inhibitory effect on the growth of the targeted tumor or infectious lesion. For purpose of detecting tumors or infectious lesions, a target binding protein or another agent is said to be administered in an "effective amount" if it can create a non-background detectable signal. For purpose of eliciting an immune response against tumors or infectious lesions, a target binding protein or another agent is said to be administered in an "effective amount" if the amount administered results in an elevated detectable immune response against at least one targeted tumor or infectious cell, preferably if it results in an elevated detectable immune response against the targeted tumor or infectious lesion, as compared to the immune response without administering said target binding protein or agent.

The present invention will be understood more readily by reference to the following examples, which are provided by way of illustration and are not intended to be limiting of the present invention.

Example 1: Construction of the DNA Sequences Encoding a Multivalent Binding Protein Comprising a hMN14 Fab Molecule Carrying Two ScFv For 734, One Fused to the C-terminal of the Kappa Chain, the Other to the C-terminal End of the Fd sequence.

As used herein, DTPA denotes diethylenetriaminepentaacetic acid. hMN-14 represents a humanized monoclonal antibody MN-14. hMN-14 is described in US Patent No. 5,874,540, which is incorporated herein by reference. The Fd portion of an antibody is the heavy chain portion of an antibody after pepsin digestion. The Fd portion of an antibody comprises the VH, CH1 and part of the hinge region. "734" denotes a monoclonal antibody against DTPA. Kappa chain is a type of immunoglobulin light chain.

The scFv for 734, denoted as 734scFv, is inserted in-frame at the C-terminal end of hMN14 Fd as follows:

Appropriate linker sequences necessary for the in-frame connection of the hMN14 heavy chain Fd to 734scFv were introduced into the VL and VK domains of 734, denoted as 734VL and 734VK, respectively, by PCR reactions using specific primer sets. PCR-amplification of 734VL was performed using the primer set 734VLscFv5'(Cys) and 734VLscFv3'.

The primer 734VLscFv5'(Cys) has the sequence of:

5′ TCTCTGCAGAGCCCAAATCTTGTGGTGGCGGTTCACAGCTGGTTGTGACTCAG 3′
P K S C G G G S Q L V V T Q

It represents the sense-strand sequence encoding the first four residues (PKSC) of the human IgG1 hinge, linked in-frame to the first six residues (QLVVTQ) of 734 VL, via a short flexible linker, GGGS. One Cys of the human hinge was included because it is required for the interchain disulfide linkage between the hMN14 heavy chain Fd-734scFv fusion and the hMN14 light chain. A Pst1 site was incorporated (underline) to facilitate ligation at the intronic sequence connecting the CH1 domain and the hinge.

The primer 734VLscFv3' has the sequence of:

5' AGCCTCCGCCTCCTGATCCGCACCTCCTAAGATCTTCAGTTTGGTTCC 3'
G G G G S G G G G L I K L K T G

It represents the anti-sense sequence encoding the last six residues (TKLKIL) of the 734 VL domain, and part of the flexible linker sequence (GGGGSGGGG), which is fused in-frame downstream of the VL domain.

The PCR-amplified product (^{~}400 bp) was first treated with T4 DNA polymerase to remove the extra A residue added to the termini during PCR-amplification, and was subsequently digested with Pst1. The resultant product was a double-stranded DNA fragment with a Pst1 overhang and a blunt end.

PCR amplification of 734VH was performed using the primer set 734VHscFv5' and 734VHscFv3'(Sac1).

The primer 734VHscFv5' has the sequence of:

5' CCGGAGGCGGTGGGAGTGAGGTGAAACTGCAGGAGT 3'
S G G G G S E V K L Q E

It represents the sense-strand sequence encoding the remaining part of the flexible linker sequence (SGGGGS) connecting the VL and VH sequences, and the first six residues (EVKLQE) of the 734 VH domain.

The primer 734VHscFv3'(Sac1) has the sequence of:

5'AACCTTGAGCTCGGCCGTCGCACTCATGAGGAGACGGTGACCGT 3'
* S S V T V T

It represents the anti-sense sequence encoding the last six residues (TVTVSS) of 734 VH. Also included is a translation stop codon (*). At position downstream of the stop codon, the restriction sites Eag1 (bold) and Sac1(underlined) were incorporated to facilitate subcloning.

Similarly, the PCR-amplified VH product of ^{~}400 bp was first treated with T4 DNA polymerase to remove the extra A residues at the PCR product termini, and then digested with Sac1, resulting in a VH DNA fragment with a blunt end-sticky end configuration.

A pBlueScript (Stratagene, La Jolla)-based staging vector (HC1kbpSK) containing a SacII fragment of the human IgG1 genomic sequence was constructed. The genomic SacII fragment contains a partial 5' intron, the human IgG1 CH1 domain, the intronic sequence connecting the CH1 to the hinge, the hinge sequence, the intronic sequence connecting the hinge to the CH2 domain, and part of the CH2 domain. The segment containing the hinge and part of the CH2 domain in HC1kbpSK was removed by Pstl/Sac1 digestion, and the cloning site generated was used to co-ligate the VL (Pst1/blunt) and VH (blunt/Sac1) PCR products prepared above. The CH1 domain in the resultant construct (CH1-734pSK) is connected to the 734scFv gene sequence via an intron.

Since the genomic SacII fragment for IgG1 only included part of the 5' intron sequence flanking the CH1 domain, the full intronic sequence was restored by inserting the remaining intronic sequence as a BamH1/SacII segment, into the corresponding sites of the CH1-734pSK. The BamH1/Eag1 fragment containing the full 5' intron, CH1 domain, connecting intron, 5 hinge-residues, short GGGS linker, and a 734scFv sequences was then isolated, and used to replace the HindIII/Eag1 segment containing the human genomic IgG1 constant sequence in the hMN14pdHL2 vector. The hMN14pdHL2 vector was described in Leung SO, Losman MJ, Qu Z, Goldenberg DM and Hansen HJ, Enhanced Production of a Humanized Anti-carcinoembryonic Antigen Antibody, Tumor Targeting 2:184(#95) (1996). For pdHL2 vector, please see Losman MJ, Qu Z, Krishnan IS, Wang J, Hansen HJ, Goldenberg DM and Leung SO, Generation and Monitoring of cell lines producing humanized antibodies, Clin. Cancer Res., 5:3101s-3105s (1999), and Losman MJ, Hansen HJ, Dworak H, Krishnan IS, Qu Z, Shih LB, Zeng L, Goldenberg DM and Leung SO, Generation of a high-producing clone of a humanized anti-B-cell lymphoma monoclonal antibody (hLL2), Cancer (suppl), 80:2660-2666 (1997). These references, as well as any cited references in this disclosure, are hereby incorporated by reference.

A HNB linker with a BamH1 overhang on one end and a HindIII overhang on the other was used to facilitate the BamH1/Eag1 fragment ligation into the HindIII/Eag1 site in the hMN14pdHL2 vector. It has the sequence of:

5′ AGCTTGCGGCCGC 3′
3′ ACGCCGGCGCTAG 5′

The resultant vector is designated as hMN14-734pdHL2.

To insert a 734 scFv to the C-terminal end of the kappa chain for hMN-14 Fab, a similar strategy is used and described as follows:

A Sac1 fragment containing part of the 5' intron flanking the human CK domain, and most of the CK region sequence was co-ligated into the Sac1/BamH1 cloning site of a pBlueScript vector in the presence of a linker, CKSB. The CKSB linker contains two synthetic DNA nucleotide, which, when annealed, will generate a double stranded DNA encoding the last 13 amino acid of the human CK region, fused in-framed to the first 4 residues of the human IgG1 hinge, at the C-terminal of which attached a short flexible linker (GGGS). The CKSB linker has the double-stranded sequence of:

5′ CGCCCGTCACAAAGAGCTTCAACAGGGGAGAGTGTGAGCCCAAATCTGGTGGCG 3′
3′ TCGAGCGGGCAGTGTTTCTCGAAGTTGTCCCCTCTCACACTCGGGTTTAGACCACCGCCTAG 5′
S P V T K S F N R G E C E P K S G G G S

The Sac1 3' overhang of the CKSB linker will ligate to the C-terminal Sac1 of the CK fragment, while the BamH1 end will ligate to the corresponding BamH1 site of the pBlueScript vector. The resultant staging vector is designated as CK(B)pSK.

The VL region of 734 was PCR-amplified with the primer set 734VLscFv5'(BglII) and 734VLscFv3'. The primer 734VLscFv5' (BglII) has the sequence of:

5′ TCTAGATCTCAGCTGGTTGTGACTCAG 3′
S Q L V V T Q

It represents the sense-strand sequence encoding the first six residues (QLVVTQ) of 734 VL. A 5' BglII site was incorporated (underlined) to facilitate subsequent ligation to the short flexible linker connecting to the CK domain.

The sequence of the 734VLscFv3' has been previously described.

The PCR-amplified product for 734 VL was treated with T4 DNA polymerase and BglII, generating a blunt end/BglII sticky end fragment.

PCR-amplification of 734 VH was performed using the primer set 734VHscFv5' and 734VHscFv3'(Sall).

The sequence of 734VHscFv5' has been described previously.

The sequence of 734VHscFv3'(Sal1) is basically the same as 734VHscFv3'(Sac1) except that the Sac1 site was replaced by Sal1 (underlined).

5′ AACCCTTGTCGACGGCCGTCGCACTCATGAGGAGACGGTGACCGT 3′
* S S V T V T

Similarly, the PCR-amplified VH product was first treated with T4 DNA polymerase to generate a blunt end and digested with Sal1 to generate a sticky end.
The staging vector CK(B)pSK was digested with BamH1 and Sal1, and the exposed cloning sites were inserted with the VL and VH PCR product. The BglII overhang of VL is complementary to the BamH1 overhang at the C-terminal end of the CK-fragment in the digested vector, whereas the Sal1 end is ligated to the corresponding site in the downstream end of the vector. The resultant vector, designated as CK-734scFvpSK, carries the genomic CK sequence fused via a short peptide to the 734scFv sequence.

The CK-734scFvpSK vector was first linearized with HincII enzyme which cuts at the Sal1 site to generate a blunt end, and the CK-734scFv fragment was cut out by partial digestion with Sac1. The fragment containing the CK-734scFv gene sequence with size of about 1.3 Kb was isolated and ligated at the Sac1/PflM1 site of the hMN14pdHL2 vector, replacing the original CK sequence. The PflM1 site is located at the 3' non-coding sequence about 50 bp downstream of the CK stop codon, and the 3' overhang generated by PflM1 digestion was filled in with Klenow enzyme before the CK-734scFv fragment was ligated into the hMN14-734pdHL2 vector. The final expression vector, designated as hMN14-Di-734pdHL2, encodes a hMN14 Fab molecule carrying two scFv for 734, one fused to the C-terminal of the kappa chain, the other to the C-terminal end of the Fd sequence.

### Example 2: Expression and Purification of a Trivalent Bi-specific Antibody Containing a CEA Specific Fab Linked With Two Anti-DTPA ScFv Derived From the Antibody 734

To construct a trivalent BI-specific antibody containing a CEA specific Fab linked with two anti-DTPA scFv derived from the antibody 734, a 734 scFv is fused to the C-terminal end of the hMN-14 Fd sequence to form a Fd-scFv gene sequence; and the same 734 scFv sequence will be attached to the C-terminal end of the hMN-14 kappa chain sequence, forming a kappa-scFv gene sequence. The Fd-scFv and kappa-scFv sequences will be assembled in the expression vector pdHL2. The resultant expression vector, designated as hMN14-di-scFv734pdHL2, will be used to transfect SP2/0 cells by electroporation using standard procedures. The expression vector contains a DHFR gene and can be used as the selection marker for transfected cells using 0.1 µM of methotrexate (MTX). ELISA assay can be used for the detection of antibody secreting clones, which are subsequently amplified by step-wise increase in the MTX concentration. The secreted antibody can be purified by protein G affinity column. Further purification can be achieved with ion-exchange chromatography.

## Claims

1. A target binding protein, comprising
(a) a first polypeptide comprising a first single chain Fv binding site (scFv) joined by a first peptide linker to the CL of a VL-CL immunoglobulin domain and
(b) a second polypeptide comprising a second single chain Fv binding site (scFv) joined by a second peptide linker to the CH1 of a VH-CH1 immunoglobulin domain, wherein the CH1 is human IgG1 CH1,
wherein each of the first and second scFv forms a target binding site independently, and
wherein the VL region and VH region associate to form a target binding site; and
wherein the cysteine residue in the second peptide linker forms a disulfide bond with the CL region.

2. The target binding protein of claim 1, wherein the first peptide linker has the amino acid sequence GGGS and wherein the second peptide linker has the amino acid sequence EPKSCGGGS.

3. The target binding protein of claim 1, wherein the three binding sites have the same target binding specificity.

4. The target binding protein of claim 1, wherein two of the binding sites have the same target binding specificity.

5. The target binding protein of claim 1, wherein the three binding sites each have a different target binding specificity from each other.

6. The target binding protein of any one of claims 1 to 5, which comprises humanized variable region domains.

## Patentansprüche

1. Zielbindendes Protein, umfassend
(a) ein erstes Polypeptid umfassend eine erste Einzelketten-Fv-Bindungsstelle (scFv), die durch einen ersten Peptidlinker mit der CL einer VL-CL-Immunglobulin-Domäne verbunden ist, und
(b) ein zweites Polypeptid umfassend eine zweite Einzelketten-Fv-Bindungsstelle (scFv), die durch einen zweiten Peptidlinker mit der CH1 einer VH-CH1-Immunglobulin-Domäne verbunden ist, wobei die CH1 eine menschliche IgG1-CH1 ist,
wobei eine jede der ersten und zweiten scFv unabhängig eine Zielbindungsstelle bildet und wobei die VL-Region und VH-Region assoziieren, um eine Zielbindungsstelle zu bilden; und
wobei der Cysteinrest in dem zweiten Peptidlinker eine Disulfidbindung mit der CL-Region ausbildet.

2. Zielbindendes Protein nach Anspruch 1, wobei der erste Peptidlinker die Aminosäuresequenz GGGS aufweist, und wobei der zweite Peptidlinker die Aminosäuresequenz EPKSCGGGS aufweist.

3. Zielbindendes Protein nach Anspruch 1, wobei die drei Bindungsstellen die gleiche Zielbindungsspezifität aufweisen.

4. Zielbindendes Protein nach Anspruch 1, wobei zwei der Bindungsstellen die gleiche Zielbindungsspezifität aufweisen.

5. Zielbindendes Protein nach Anspruch 1, wobei die drei Bindungsstellen jeweils eine voneinander verschiedene Zielbindungsspezifität aufweisen.

6. Zielbindendes Protein nach einem der Ansprüche 1 bis 5, welches Domänen der humanisierten variablen Region umfasst.

## Revendications

1. Protéine de liaison à une cible, comprenant
(a) un premier polypeptide comprenant un premier site de liaison à des Fv monocaténaires (scFv), réuni par un premier lieur peptidique à la CL d'un domaine VL-CL d'immunoglobuline, et
(b) un deuxième polypeptide comprenant un deuxième site de liaison à des Fv monocaténaires (scFv), réuni par un deuxième lieur peptidique à la CH1 d'un domaine VH-CH1 d'immunoglobuline, la CH1 étant une IgG1 CH1 humaine,
chacun des premiers et des deuxièmes scFv formant d'une manière indépendante un site de liaison à une cible, et la région VL et la région VH s'associant pour former un site de liaison à une cible ; et le résidu cystéine du deuxième lieur peptidique formant une liaison disulfure avec la région CL.

2. Protéine de liaison à une cible de la revendication 1, dans laquelle le premier lieur peptidique a la séquence d'acides aminés GGGS, et le deuxième lieur peptidique a la séquence d'acides aminés EPKSCGGGS.

3. Site de liaison à une cible de la revendication 1, dans lequel les trois sites de liaison ont la même spécificité de liaison à une cible.

4. Protéine de liaison à une cible de la revendication 1, dans laquelle deux des sites de liaison ont la même spécificité de liaison à une cible.

5. Protéine de liaison à une cible de la revendication 1, dans laquelle les trois sites de liaison ont des spécificités de liaison à une cible qui sont différentes l'une de l'autre.

6. Protéine de liaison à une cible de l'une quelconque des revendications 1 à 5, qui comprend des domaines de régions variables humanisés.
